# EUROPÄISCHE PATENTSCHRIFT

(11) **EP 1 988 858 B1**
(45) Veröffentlichungstag und Bekanntmachung des Hinweises auf die Patenterteilung: **22.02.2012**
(21) Anmeldenummer: 07711204.3
(22) Anmeldetag: 22.02.2007
(51) Int. Cl.: A61F 2/60, A61F 2/68

(54) **HÜFTGELENKPROTHESE**
HIP JOINT PROSTHESIS
PROTHÈSE DE HANCHE

(30) Priorität: 27.02.2006 DE 102006009510
(43) Veröffentlichungstag der Anmeldung: 12.11.2008
(73) Patentinhaber: Otto Bock HealthCare GmbH, 37115 Duderstadt (DE)
(72) Erfinder: BOITEN, Herman, 37085 Göttingen (DE)
(74) Vertreter: Stornebel, Kai
(86) Internationale Anmeldenummer: PCT/DE2007/000351
(87) Internationale Veröffentlichungsnummer: WO 2007/095933

(56) Entgegenhaltungen:
- WO-A-01/17466
- DE-A1- 19 621 034
- DE-C1- 19 935 203
- DE-U1- 9 320 853
- US-A- 4 051 558
- US-A- 5 746 774

## Beschreibung

Die Erfindung betrifft eine Hüftgelenkprothese für ein Kunstbein mit Anschlussmitteln zur Befestigung an einer oberen Befestigungseinrichtung und einem sich an der Hüftgelenkprothese anschließenden Kunstbein. Die Erfindung ist insbesondere bei einer Exartikulation des Hüftgelenkes einsetzbar.

Das Gehen mit einer Hüftgelenkprothese mit einem daran befestigten Kunstbein ist schwierig, da drei Gelenke, nämlich das Hüftgelenk, das Kniegelenk und das Fussgelenk gesteuert werden müssen, ohne dass der Prothesenträger eine Möglichkeit zur aktiven Einflussnahme auf diese Gelenke hat. Insbesondere ist die Steuerung der Bewegungen des Hüftgelenkes für die Fortbewegung und für die Stabilität in der Standphase wichtig.

Um sicherzustellen, dass nach dem Fersenstoß das Hüftgelenk eine Streckung ausführt, werden die Gelenkachsen heutiger Hüftgelenkprothesen anterior zu den Gelenkachsen eines natürlichen Hüftgelenkes angeordnet. Dieses Konzept ist in der US 3,090,964 A1 beschrieben, in der ein einstellbares Hüftgelenk beschrieben ist, um einen dynamischen Aufbau bereitstellen zu können. Die vorgeschlagene Anordnung stellt sicher, dass eine maximal gestreckte Stellung des Hüftgelenkes kurz nach dem Fersenstoß eingenommen wird.

Die US 4,215,441 beschreibt eine an einem Beckenkorb befestigte Beinprothese mit einer Feder, die während der Standphase durch eine axial wirkende Kraft, z. B. die Kraft aufgrund des Gewichtes des Prothesennutzers, vorgespannt wird. Beugt sich jetzt das Prothesenkniegelenk, entspannt sich die Feder und bewirkt eine Beugung im Hüftgelenk und in Folge dessen eine weitere Beugung des Kniegelenkes und eine Vorwärtsbewegung des Prothesenfußes. Ein ähnlicher Mechanismus ist in der US 4,051,558 beschrieben.

Aus der WO 01/17466 A2 ist eine Beinprothese mit einer pneumatischen Energiespeichereinheit bekannt. Während der Streckbewegung in dem Hüftgelenk wird En-ergie gespeichert, die während der darauf folgenden Beugebewegung wieder abgegeben wird. Die Energiespeichereinrichtung weist eine elektronisch gesteuerte Kolben/-Zylindereinheit auf, wobei die Kolbenstange teleskopierbar ist, um die Beugung des Hüftgelenks der Schwingphase zu begrenzen. Um einen stabilen und sicheren Fersenauftritt zu gewährleisten, wird eine kurze Schrittlänge gewählt.

Die bekannten Vorrichtungen zur Begrenzung der Schrittlänge benötigen eine manuelle Betätigung, um eine weitergehende Beugung zu ermöglichen, beispielsweise um sich zu setzen.

Mit den aus dem Stand der Technik bekannten Prothesen ist die Erzielung eines möglichst natürlichen Gangbildes nicht möglich. Eine schnelle Streckung des Hüftgelenkes nach dem Fersenstoß ist keine natürliche Bewegung. Die Bewegung ist für den Patienten nicht kontrollierbar, da ein Balancieren auf dem vor der Schwerkraftlinie liegenden freien Gelenk nicht möglich ist. Das natürliche Abheben und Durchschwingen des kontralateralen Beines während der Streckung des künstlichen Hüftgelenkes ist bei Hüftgelenken aus dem bekannten Stand der Technik ausgeschlossen. Mit einer fixierten Schrittlänge ist es nicht möglich, verschiedene Gehgeschwindigkeiten aufgrund der damit einhergehend erforderlichen verschiedenen Schrittlängen zu ermöglichen. Dabei muss bei einer variablen Schrittlänge, die abhängig von der Gehgeschwindigkeit des Prothesennutzers ist, ein sicherer und stabiler Fersenauftritt gewährleistet sein, ohne das Risiko einer weitergehenden, unkontrollierten Hüftbeugung in Kauf nehmen zu müssen. Eine Dämpfung sowohl der Streck- als auch der Beugebewegung ist im Stand der Technik nicht vorgesehen.

Aufgabe der vorliegenden Erfindung ist es, eine Hüftgelenkprothese bereitzustellen, mit der die oben genannten Probleme vermieden oder minimiert werden können.

Erfindungsgemäß wird dies durch eine Hüftgelenkprothese mit den Merkmalen des Anspruchs 1 und ein Steuerungsverfahren nach den Merkmalen des Anspruchs 9 gelöst. Vorteilhafte Ausgestaltungen und Weiterbildungen der Erfindung sind in den Unteransprüchen beschrieben.

Die erfindungsgemäße Hüftgelenkprothese weist Anschlussmittel zur Befestigung an einer Befestigungseinrichtung und für ein Kunstbein auf. Darüber hinaus ist eine Steuereinheit zur Steuerung einer Streckbewegung im Hüftgelenk und der Schrittlänge vorgesehen. Mit der Steuereinheit ist es möglich, die Streckbewegung in der Standphase und die Schrittlänge der Beinprothese zu steuern, beispielsweise über ein hydraulisches Steuersystem. Die Erfindung sieht weiterhin vor, dass die Steuereinheit zumindest eine Dämpfereinrichtung zur Dämpfung der Beuge- und/oder der Streckbewegung in dem Hüftgelenk aufweist, um die Schrittlänge und die Art der Hüftbeugung oder Hüftstreckung kontrollieren zu können.

Für die Beugebewegung ist es vorgesehen, dass über die Einstellvorrichtungen oder - mittel ein erster Bewegungsabschnitt definiert ist, in dem keine oder nur eine sehr geringe Dämpfung vorhanden ist. In einem zweiten Bewegungsabschnitt steigt die Dämpfung an, bevorzugt progressiv, bis auf einen festgelegten Grenzwert, der in Abhängigkeit von biometrischen Daten des Prothesennutzers sowie dessen Wünschen eingestellt werden kann. In einem daran sich anschließenden dritten Bewegungsabschnitt bleibt die Dämpfung konstant auf dem Endniveau des zweiten Bewegungsabschnittes und sichert einen stabilen Fersenauftritt ohne die Gefahr einer weiteren, unkontrollierten Hüftbeugung. Der Beugewinkel, also die Länge des jeweiligen Bewegungsabschnittes, kann eingestellt werden, so dass je nach individuellen Wünschen der nahezu ungedämpfte erste Bewegungsabschnitt verlängert oder verkürzt werden kann. Dementsprechend kann der zweite Bewegungsabschnitt mit ansteigender Dämpferkennlinie in der Steigung der Dämpferkennlinie variabel gestaltet sein, um beispielsweise die Schrittlänge zu verkürzen, wenn ein Prothesennutzer ein hohes Bedürfnis an Stabilität hat. Andererseits können die jeweiligen Bereiche auch so gewählt werden, dass größere Schrittlängen zugelassen sind, um den Bedürfnissen aktiver Prothesennutzer gerecht zu werden.

Die Dämpfereinrichtung ist bevorzugt als ein hydraulischer Dämpfer ausgebildet und weist Vorrichtungen oder Mittel zum Einstellen der Dämpfung in der Streckrichtung der Prothese und der Beugerichtung der Prothese auf. Bevorzugt ist die Steuereinheit mit einer entsprechenden Dämpferkennlinie ausgestattet, so dass bei einer erhöhten Gehgeschwindigkeit mit entsprechend höherem Energieniveau eine an das natürliche Gangbild angepasste Schrittlängenvergrößerung stattfindet.

Die Steuereinheit ist bevorzugt in dem Hüftgelenk integriert, um eine kompakte Bauweise bereitzustellen und weist bevorzugt einstellbare Ventile oder Widerstände auf, um die Dämpferkennlinie variabel gestalten zu können.

Für ein besonders natürliches Gangbild sind Einstellvorrichtungen oder -mittel zur Einstellung der Dämpfung in der Streckung und Beugung vorgesehen. In der Streckung ist eine hohe Dämpfung vorgesehen, die so eingestellt werden kann, dass das Becken des Patienten beim Abrollen auf der Prothese nahezu horizontal verbleibt. Eine plötzliche Streckung nach dem Fersenstoß wird vermieden. Dadurch wird es dem Patienten ermöglicht, während der Streckung des künstlichen Hüftgelenkes das Bein auf der kontralateralen Seite anzuheben und durchzuschwingen. Die Höhe der Dämpfung kann eingestellt werden, so dass je nach individuellen Wünschen eine schnellere oder langsamere Streckung möglich ist. Wenn ein hohes Sicherheitsbedürfnis vorliegt, kann eine schnellere Streckung eingestellt werden, wenn aber ein hohes Maß an Komfort und ein natürliches Gehen erwünscht sind, kann die Dämpfung so hoch gewählt werden, dass der Endanschlag erst kurz vor dem Abheben der Prothese erfolgt.

Die Hüftgelenkprothese kann mit einer Energiespeichereinheit versehen sein, die bei einer Streckung des Kunstbeines im Hüftgelenk Energie speichert und zur Unterstützung der Beugebewegung zumindest teilweise an das Kunstbein abgibt. Als Energiespeichereinheit sind Federelemente, insbesondere Kunststofffederelemente, Stahl- oder Karbonfedern oder andere Materialien mit entsprechenden elastischen Eigenschaften vorgesehen.

Das erfindungsgemäße Verfahren zur Steuerung einer Hüftgelenkprothese mit zumindest einer Dämpfereinheit zur Dämpfung der Beuge- und Streckbewegung eines Kunstbeines sieht vor, dass die Dämpfung der Streckbewegung so eingestellt wird, dass ein Streckanschlag noch nicht erreicht wird, wenn bei einem normalen Gangmuster das Bein der contralateralen Seite schon angehoben wird. Die notwendige Dämpfung der Streckbewegung kann auf den jeweiligen Patienten abgestimmt, eingestellt werden. Dadurch ist es möglich, Vorlieben der jeweiligen Prothesenträger zu berücksichtigen und die Hüftgelenkprothese an das jeweilige Gangbild anzupassen. Ergänzend oder alternativ wird die Steuerung so ausgeführt, dass in einem ersten Bewegungsabschnitt die Beugung nicht gedämpft wird, also dass ohne einen erhöhten Bewegungswiderstand eine Beugung stattfinden kann, so dass hierdurch die Schrittlänge aufgrund des Beugewinkels eingestellt werden kann. Während des zweiten Bewegungsabschnittes wird die Dämpfung erhöht, entweder linear oder progressiv, um dann in einem dritten Bewegungsabschnitt konstant auf einem Endniveau gehalten zu werden, das dem Endniveau der Dämpfung des zweiten Bewegungsabschnittes entspricht.

Somit kann eine Steuerung der Standphasendämpfung in Kombination zur Steuerung der Schwungphasendämpfung oder separat dazu ausgeführt werden, um eine an das natürliche Gangbild angepasste Bewegung zu ermöglichen.

Eine Weiterbildung der Erfindung sieht vor, dass als Steuerungsparameter für die Dämpfereinheit der Beugewinkel, die Bewegungsrichtung, die Beugegeschwindigkeit des Kunstbeines und/oder die auf das Kunstbein ausgeübten Kräfte gemessen werden. In Abhängigkeit von den gemessenen Steuerungsparametern erfolgt dann eine automatische Anpassung der Dämpfung bzw. der Dämpfungskennlinie in dem jeweils gewünschten Maße. Die Steuerungsparameter werden dabei über Sensoren ermittelt und einer elektronischen Auswerteeinheit zugeführt, die auf der Grundlage der ermittelten Werte eine Verstellung der Dämpfereinheiten, beispielsweise Ventile, über Aktuatoren veranlasst.

Nachfolgend wird ein Ausführungsbeispiel der Erfindung anhand der Figuren näher erläutert. Es zeigen:
- Figur 1 -: eine Hüftgelenkprothese in perspektivischer Teilschnittdarstellung;
- Figur 2 -: eine Hüftgelenkprothese in Schnittdarstellung in leichter Beugung;
- Figur 3 -: eine Detailansicht der Figur 2;
- Figur 4 -: eine Hüftgelenkprothese in einer gebeugten Stellung in Seitenansicht;
- Figur 5 -: eine Hüftgelenkprothese in einer gestreckten Stellung;
- Figuren 6 bis 9 -: Schaltbilder einer Hüftgelenkshydraulik; sowie
- Figur 10 -: ein Dämpfungskraftdiagramm für eine Schwungsphasensteuerung.

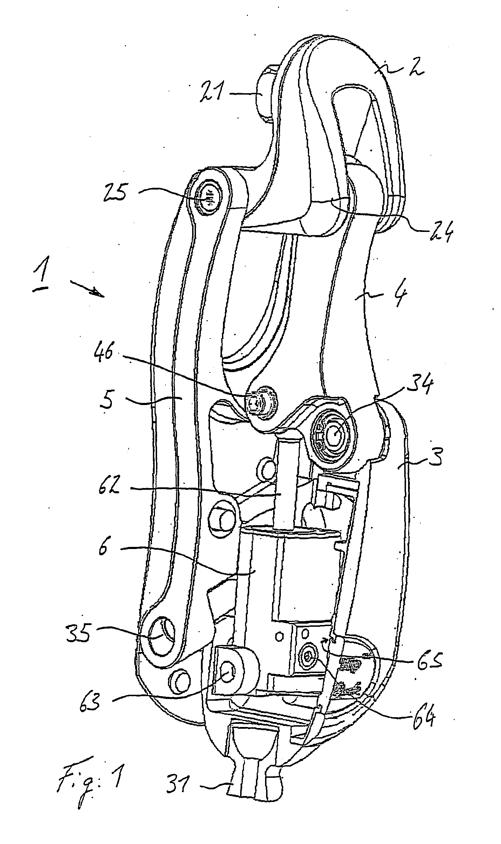

In der Figur 1 ist eine Hüftgelenkprothese 1 in perspektivischer Teilschnittdarstellung gezeigt, mit einem Oberteil 2 und einem teilgeschnittenen Unterteil 3, die über einen vorderen Lenker 4 und einem hinteren Lenker 5 gelenkig miteinander verbunden sind. An dem Oberteil 2 ist ein oberer Anschluss 21 zur Befestigung an einem Prothesenkorb oder dergleichen befestigt. Über eine Drehachse 25 ist das Oberteil 2 gelenkig mit dem hinteren Lenker 5 verbunden. Über ein Kugelgelenk 24 ist das Oberteil 2 gelenkig mit dem vorderen Lenker 4 verbunden. Der vordere Lenker 4 ist mit einem unteren Kugelgelenk 34 an dem Unterteil 3 verbunden, während der hintere Lenker 5 mit seiner unteren Drehachse 35 an dem Unterteil 3 gelagert ist.

An dem vorderen Lenker 4 ist zum Gelenkinneren, also zum hinteren Lenker 5 gewandt, eine Lagerstelle für eine Kolbenstange 62 ausgebildet, die verschwenkbar um ein Kugelgelenk 46 gelagert ist. Aufgrund des Abstandes des unteren Kugelgelenkes 34 zu dem Kugelgelenk 46 der Kolbenstange 62 findet bei einer Verschwenkung des vorderen Lenkers 4 um das untere Kugelgelenk 34 neben einer Drehung relativ zu der Kolbenstange 62 auch eine Verschiebung an der Kolbenstange 62 statt, so dass diese in Abhängigkeit von der Drehrichtung des vorderen Lenkers 4 hin- und herbewegt wird.

Die Kolbenstange 62 ragt in das Unterteil 3 hinein und wird dort von einer Steuereinheit 6 aufgenommen, die in dem Unterteil 3 drehbar um die Drehachse 63 gelagert ist. An dem unteren Ende des Unterteils 3 ist ein unterer Anschluss 31 zur Verbindung mit einem Kunstbein befestigt.

In dem Unterteil 3 ist die Steuereinheit 6 um eine Schwenkachse 63 beweglich gehalten. Die Schwenkachse 63 der Steuereinheit 6 liegt am unteren Ende der Steuereinheit 6 und verhindert ein Verkanten eines innerhalb der Steuereinheit 6 an der Kolbenstange 62 geführten Kolbens, das bei einer starren Lagerung der Steuereinheit 6 aufgrund der Bewegungskomponente senkrecht zur Kolbenstangenausrichtung auftreten würde. Diese zweite Bewegungskomponente senkrecht zur Kolbenstange 62 ist aufgrund der Schwenkbewegung um das untere Kugelgelenk 34 des vorderen Lenkers 4 und der dadurch ausgeführten Kreisabschnittsbewegung der Schwenkachse 46 der Kolbenstange 62 gegeben.

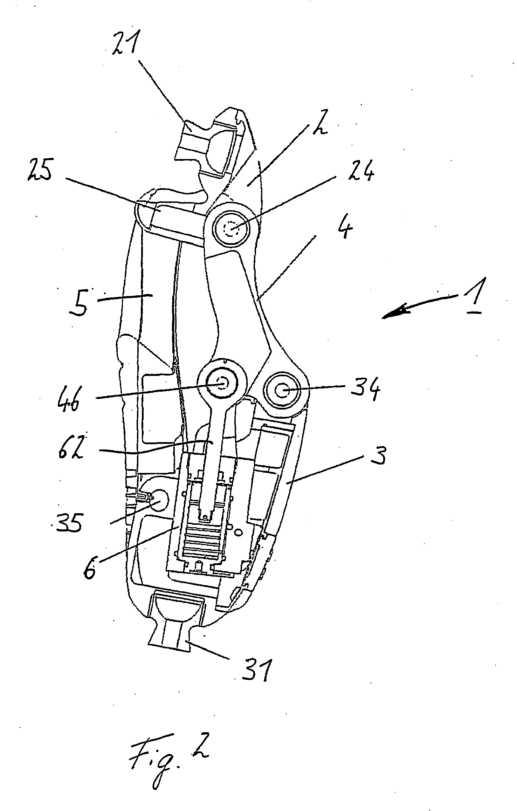

Figur 2 zeigt die Hüftgelenkprothese 1 in Schnittdarstellung in Gesamtdarstellung.

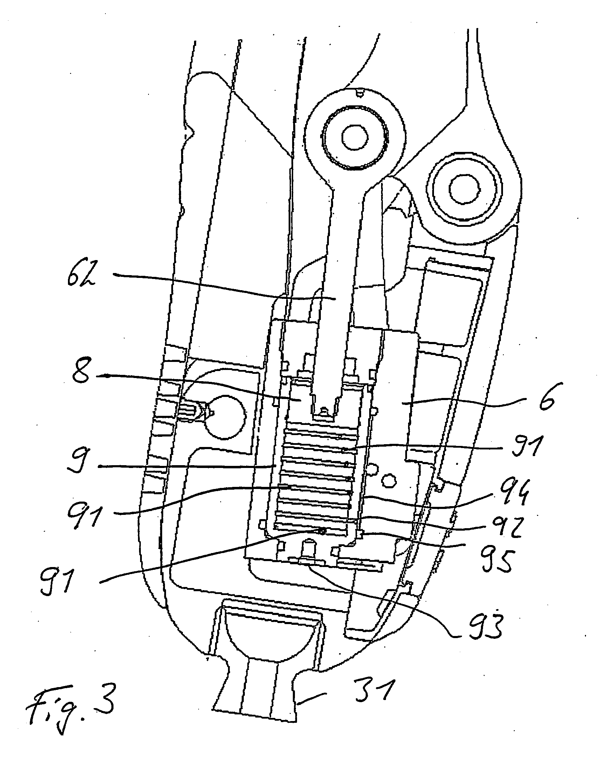

In der Figur 3 ist die Hüftgelenkprothese 1 in vergrößerter Detaildarstellung in einer leicht gebeugten Stellung gezeigt, bei der der Kolben 8 am oberen Ende des durch die Zylinderwandung 9 ausgebildeten Zylinderraumes angeordnet ist.

In der Figur 3 sind die in Nuten 91 angeordneten, in Umfangsrichtung versetzt zueinander ausgerichteten Bohrungen 92 zu erkennen, die jeweils in Ausrichtung zu dem Rückführkanal 94 gebracht werden können. Die unterste Nut 91 steht in ständiger Verbindung mit einem Ringkanal 95 und bildet den minimalen Hydraulikrückstrom aus, der stets gewährleistet ist. Dadurch wird die Dämpfung konstant auf einem Endniveau gehalten. Bis zum Erreichen derjenigen Nut 91, die ebenfalls mit dem Rückführkanal 94 über eine Bohrung 92 in Verbindung steht, wird ein entsprechend verringertes Dämpfungsniveau aufgrund der höheren Rückströmmenge der durch die beiden Bohrungen 92 durchströmenden Hydraulikflüssigkeit gewährleistet. Sofern der Kolben 8 die Bohrung 92 erreicht, die mit dem Rückführkanal 94 kommuniziert, wird diese sukzessiv verschlossen, so dass sich eine ansteigende Dämpferkennlinie für diesen zweiten Bereich ergibt. Durch diese einstellbare Dämpfung in der Steuereinheit 6 ist es möglich, die Streckbewegung im Hüftgelenk zu steuern. Durch die Anordnung eines Federelementes 7 und die Einstellbarkeit der Dämpfung in der Beugebewegung ist es möglich, die Schrittlänge einzustellen.

Die Steuereinheit 6 ist eine separat ausgebildete, verschwenkbar gelagerte Hydraulikdämpfereinheit, die im Unterteil 3 des Hüftgelenks 1 integriert untergebracht ist. Eine alternative Anordnung der Steuer- oder Dämpfereinheit 6 an einem anderen Ort des Hüftgelenkes 1 ist ebenfalls möglich. Durch die externe Zugänglichkeit der Ventile 64, 65 können die jeweiligen Dämpferkennlinien und die Bewegungsabschnitte individuell eingestellt werden. Dies gilt insbesondere auch durch die Verstellbarkeit der Anordnung der Bohrungen 92.

Die Funktionsweise der Steuereinheit 6 ist am besten anhand der vergrößerten Darstellung in der Figur 3 zu erläutern. An der Kolbenstange 62 ist an dem unteren Ende der Kolben 8 befestigt. Innerhalb der Steuereinheit 6 ist die Zylinderinnenwandung 9 drehbar gelagert ausgebildet und weist die Nuten 91 auf. Jede Nut 91 besitzt eine dazugehörige Öffnung 92, die durch Verdrehen der Zylinderwandung 9 mittels eines Werkzeuges verdreht werden kann, das am unteren Ende in eine Ausnehmung 93 eingreift, beispielsweise in einen Schlitz oder einen Innensechskant, bis die Bohrung 92 mit einem vertikal ausgerichteten Rückführkanal 94 fluchtet. Über ein Verdrehen der Zylinderwandung 9 wird bestimmt, welche Bohrung 92 mit dem Rückführkanal 94 fluchtet und geöffnet wird und somit, bis zu welcher Nut 91 sich der Kolben 8 in der Beugung frei bewegen kann. Wird die Hüftgelenkprothese 1 gebeugt, bewegt sich die Kolbenstange 62 nach unten, wird das Hüftgelenk gestreckt, bewegt sich der Kolben 8 entsprechend nach oben. Die Ausnehmung 93 ist von außen durch eine Zugangsöffnung zugänglich, so dass eine Einstellung des Beugewinkels in Abhängigkeit von der Stellung der Zylinderwandung 9 und der jeweils fluchtenden Bohrung 92 mit dem Rückführkanal 94 erfolgen kann.

In den Kolben 8 kann eine Nut gefräst sein, die nach Erreichen der jeweiligen Nut 91 in der Zylinderwandung einen progressiven Dämpfungsanstieg bewirkt. Nachdem die Kolbennut komplett vor die Zylinderwandungsnut 91 gefahren ist, wirkt die hohe Dämpfung, die mit einem Extensionsventil 64 eingestellt werden kann.

Die Streckung wird mit einer konstanten oder leicht ansteigenden Dämpfung gemindert. Die Dämpfung kann mit dem zweiten Ventil 65 an der Hydraulik eingestellt werden.

Die Steuereinheit 6 ist somit als ein hydraulischer Dämpfer ausgebildet, der eine progressive Dämpferkennlinie aufweist und sowohl die Beuge- als auch die Streckbewegung in dem Hüftgelenk dämpfen und den Beugewinkel und die Streckbewegung über Ventile 64, 65 mechanisch steuern kann. Bis zum Erreichen der jeweiligen Bohrung 92, die mit dem Rückführkanal 94 fluchtet, ist kein oder nur eine minimale Dämpfung vorhanden, während ab Erreichen des Kolbens 8 der Zylinderwandungsnut 91 die Dämpfung ansteigt. Ab Erreichen und vollständigem Verschließen der Bohrung ist eine hohe Dämpfung durch die Bohrung 92 in der letzten Nut 91 vorhanden, die bis zum Ende der Beugebewegung konstant bleibt. In der Streckung fließt das Öl durch eine in dieser Figur nicht dargestellte obere Bohrung 92 in die obere Nut 91, wobei die Dämpfungskennlinie über das Ventil 65 oder eine Drossel einstellbar ist.

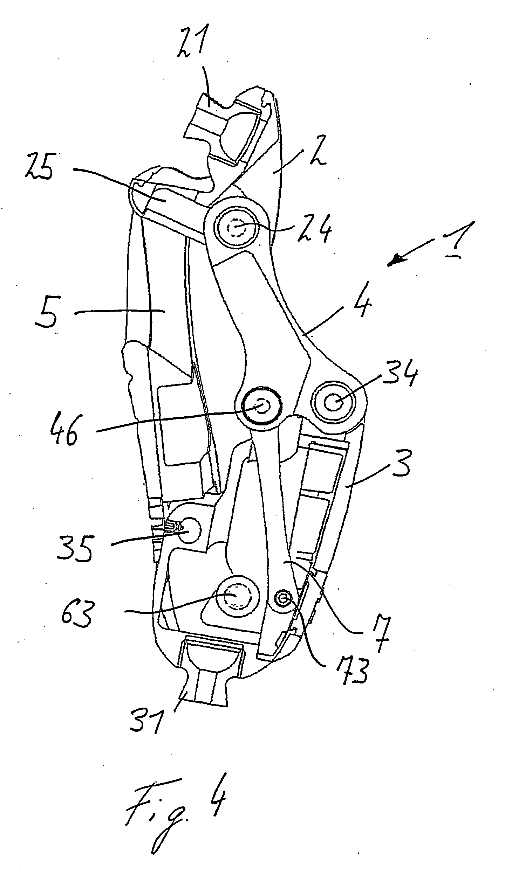

In der Figur 4 ist die Hüftgelenkprothese 1 in einer gebeugten Stellung gezeigt. In dieser Figur ist zu erkennen, dass das Kugelgelenk 46 der Kolbenstange 62 gleichzeitig einen Anlenkpunkt für das Federelement 7 darstellt, das mit seinem unteren Ende an dem Unterteil 3 an einer Schwenkachse 73 angeordnet ist. In der gebeugten Stellung der Hüftgelenkprothese 1 ist das Federelement 7, das insbesondere als eine Kunststofffeder oder ein anderes elastisches Band ausgebildet sein kann, kaum noch gedehnt, da sämtliche Energie, die in der Streckstellung in dem Federelement 7 gespeichert wurde, an das Unterteil 3 und damit an das nicht dargestellte Kunstbein abgegeben wurde. Das Federelement 7 unterstützt die Beugebewegung der Hüftgelenkprothese 1, indem es das Kugelgelenk 46 der Kolbenstange 62, die an dem vorderen Lenker 4 befestigt ist, nach unten in Richtung auf das Unterteil 3 mit einer Kraft belastet.

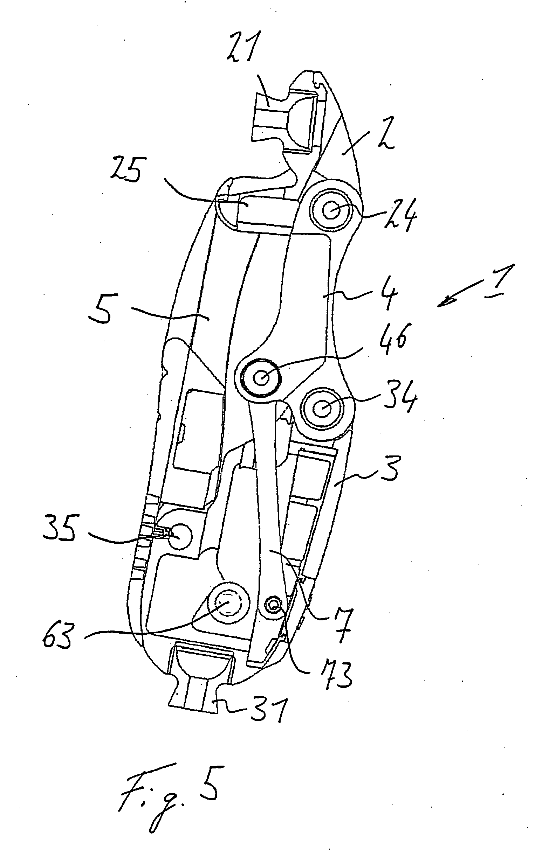

Die Streckstellung der Hüftgelenkprothese 1 ist in der Figur 5 dargestellt, die in ihrer Position im Wesentlichen der der Figur 1 entspricht. Darin ist das Federelement 7 maximal gestreckt und hat einen entsprechend hohen Energiegehalt, da der obere Anlenkpunkt 46 an dem vorderen Lenker 4 in einer maximalen Entfernung zu der unteren Schwenkachse 73 des Federelementes 7 befindlich ist. In der Federeinheit 7, die auch als herkömmliche Zugfeder ausgebildet sein kann, wird bei einer Streckung des Kunstbeines im Hüftgelenk die dabei aufgewendete Energie gespeichert und bei der Beugebewegung zumindest teilweise wieder an das Kunstbein abgegeben.

Grundsätzlich kann die Steuer- oder Dämpfereinheit nicht nur einem anderen als dem dargestellten Ort der Hüftgelenkprothese 1 angebracht werden, sondern auch bei anderen Ausgestaltungen von Hüftgelenkprothesen, beispielsweise bei einem einachsigen Hüftgelenk. Ebenfalls kann die Funktionsweise der Steuereinheit ggf. umgekehrt werden, indem bei einer Beugebewegung der Kolben ausfährt und bei einer Streckbewegung einfährt.

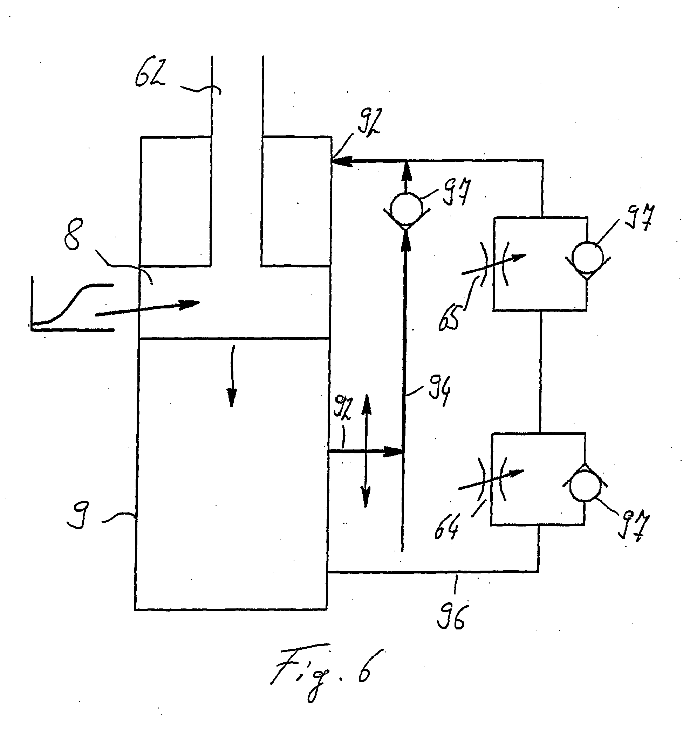

In den Figuren 6 bis 9 ist schematisch die Dämpfereinrichtung mit der Kolbenstange 62, dem Kolben 8, dem Hydraulikzylinder 9 und dem einstellbaren Beugeventil 64 und dem einstellbaren Streckventil 65 gezeigt. Die einstellbaren Ventile 64, 65 wirken als Drosselstellen, über die die Dämpfung des Hüftgelenkes eingestellt werden kann. Nicht dargestellt sind die Nuten 91 in dem Hydraulikzylinder 9. Die untere Bohrung 92 stellt die Verbindung zu dem Rückführkanal 94 dar; der der Bohrung 92 zugeordnete Doppelpfeil deutet die vertikale Einstellbarkeit und dadurch die Einstellbarkeit des Beugewinkels ohne Wirksamwerden des Beugeventils 64 an.

In der Figur 6 wird der Kolben 8 aufgrund einer Beugebewegung des Kunstbeines nach unten bewegt. Dadurch strömt die Hydraulikflüssigkeit ohne großen Widerstand durch die vertikal einstellbare Bohrung 92, über die die Schrittlängen eingestellt werden können, durch eine obere, vertikal fest eingestellte obere Bohrung 92 zurück in den oberen Teilraum des Zylinders 9. Ein Rückschlagventil 97 kann in dem Rückführkanal 94 angeordnet sein, das ein Einströmen der Hydraulikflüssigkeit in den oberen Teilraum bei einer Beugebewegung ermöglicht, ein Zurückströmen bei einer Streckbewegung jedoch verhindert.

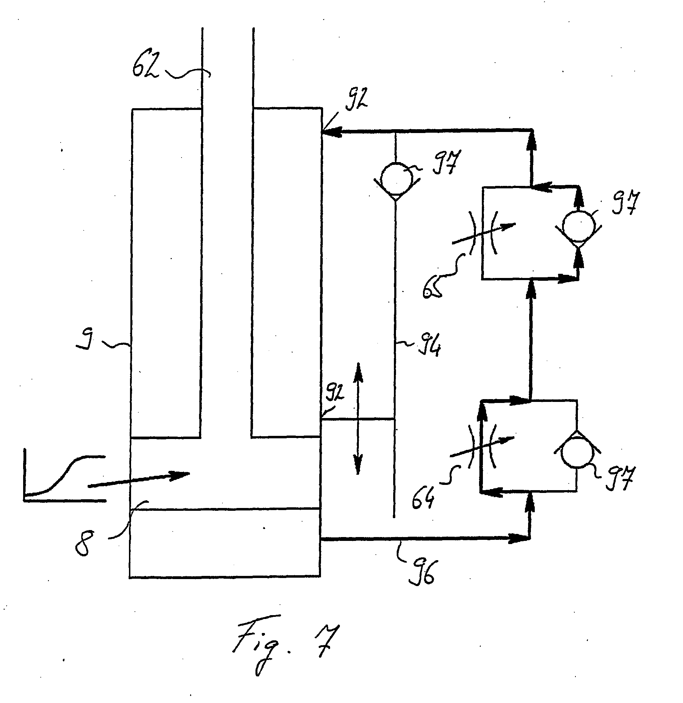

In der Figur 7 ist der Zustand dargestellt, in dem der Kolben 8 die untere Bohrung 92 erreicht und überschritten hat. Nach Erreichen der unteren Bohrung 92 strömt die Hydraulikflüssigkeit über den von dem Rückströmkanal 94 getrennten Verbindungskanal 96 zu den Ventilen 64, 65. Vor dem Erreichen der unteren Bohrung 92 und dem Verschließen durch den Kolben 8 strömte aufgrund des höheren Strömungswiderstandes in dem Verbindungskanal 96 und aufgrund der darin vorhandenen Ventile 64, 65, 97 kaum oder keine Hydraulikflüssigkeit dort hindurch. Nach dem Verschließen der vertikal einstellbaren Bohrung 92 wird die Hydraulikflüssigkeit ausschließlich durch den Verbindungskanal 96 geleitet. Durch das Beugeventil 64, das die Flexion des Hüftgelenks dämpft, wird die Dämpferkraft erhöht, so dass die Dämpferkennlinie ansteigt. Das Extensionsventil 65 wird nicht durchströmt, da ein Bypass mit einem Rückschlagventil 97 mit einem geringeren Strömungswiderstand parallel geschaltet ist.

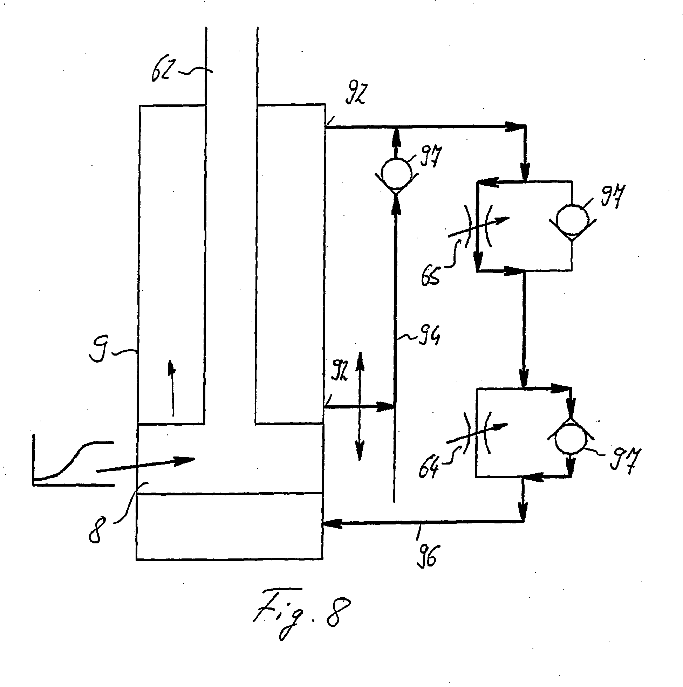

Wird nun die Streckung eingeleitet, bewegt sich der Kolben 8, wie in der Figur 8 gezeigt, aufwärts. Die Streckbewegung wird dann über das Extensionsventil 65 gedämpft, unabhängig davon, ob die Hydraulikflüssigkeit durch die obere oder untere Bohrung 92 hindurchströmt. Die Strömungsrichtung der Hydraulikflüssigkeit ist die die Pfeile angedeutet. Das Rückschlagventil 97 in dem Rückströmkanal 94 gewährleistet, so dass die gesamte Hydraulikflüssigkeit durch das Extensionsventil 65 fließt. Das Flexionsventil 64 weist einen Bypass mit einem Rückschlagventil 97 auf und hat keinen Einfluss auf die Dämpfungskennlinie in der Streckbewegung.

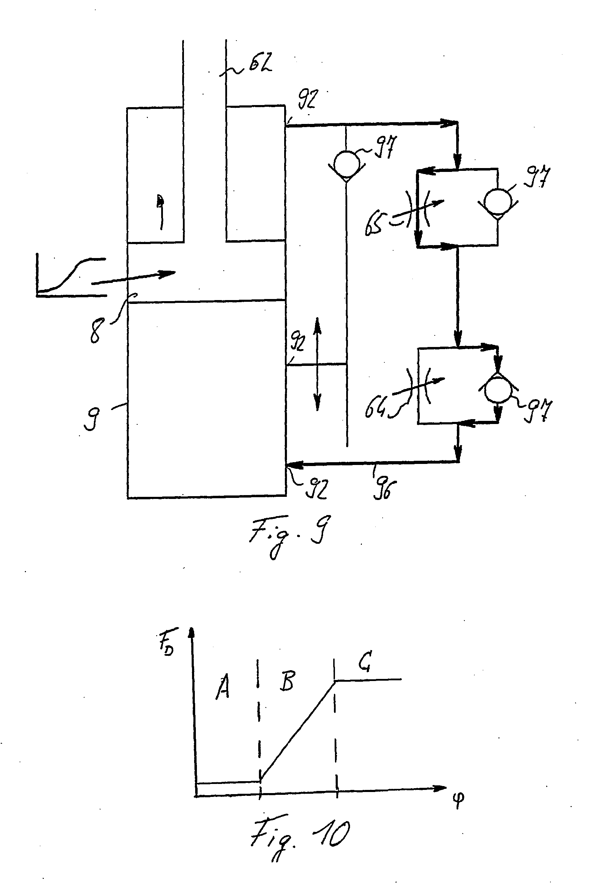

In der Figur 10 ist ein Dämpferkraftdiagramm über den Beugewinkel ϕ dargestellt, in dem die unterschiedlichen Dämpfungsphasen und wirksamen Kräfte während der Beugung gezeigt sind. In einem ersten Bewegungsabschnitt A ist die Dämpferkraft F_{D} vernachlässigbar gering und setzt sich aus den systeminhärenten Dämpfungsfaktoren zusammen, beispielsweise Reibungswiderstände an Lagerstellen oder ein stets vorhandener Strömungswiderstand der Hydraulikflüssigkeit. Eine gewollte Dämpfung findet in dem ersten Bewegungsabschnitt A nicht statt. In einem zweiten Bewegungsabschnitt B wird die Dämpfung und damit auch die als überwindende Dämpferkraft erhöht, im dargestellten Beispiel linear erhöht und zwar auf ein Endniveau, das im dritten Bewegungsabschnitt C erreicht und gehalten wird. Eine solche Dämpferkraftkurve kann durch Zuschaltung entsprechender Dämpferelemente oder Strömungswiderstände realisiert werden. Wird das Hüftgelenk nach Beendigung der Beugebewegung gestreckt, kann die Dämpfung der Streckbewegung so eingestellt werden, dass die maximale Streckung oder ein Streckanschlag noch nicht erreicht wird, wenn bei einem normalen Gangmuster, also bei normaler Gehgeschwindigkeit, das Bein der contralateralen Seite schon angehoben ist bzw. wird.

## Patentansprüche

1. Hüftgelenkprothese mit Anschlussmitteln zur Befestigung an einer oberen Befestigungseinrichtung und an einem sich an der Hüftgelenkprothese anschließenden Kunstbein sowie einer Steuereinheit (6) zur Steuerung einer Streckbewegung im Hüftgelenk und der Schrittlänge, wobei die Steuereinheit (6) zumindest eine Dämpfereinrichtung (8, 64, 65, 91, 92, 94) zur Dämpfung der Beuge- und Streckbewegung in dem Hüftgelenk aufweist, **dadurch gekennzeichnet, dass** für einen ersten Bewegungsabschnitt während der Beugung keine Dämpfung vorhanden ist, während eines zweiten Bewegungsabschnittes die Dämpfung ansteigt und in einem dritten Bewegungsabschnitt die Dämpfung konstant auf dem Endniveau des zweiten Bewegungsabschnittes bleibt.

2. Hüftgelenkprothese nach Anspruch 1, **dadurch gekennzeichnet, dass** die Steuereinheit (6) als hydraulischer Dämpfer ausgebildet ist.

3. Hüftgelenkprothese nach Anspruch 1 oder 2, **dadurch gekennzeichnet, dass** die Steuereinheit (6) eine progressive Dämpferkennlinie aufweist.

4. Hüftgelenkprothese nach einem der voranstehenden Ansprüche, **dadurch gekennzeichnet, dass** die Steuereinheit (6) in dem Hüftgelenk integriert ist.

5. Hüftgelenkprothese nach einem der voranstehenden Ansprüche, **dadurch gekennzeichnet, dass** die Dämpferkennlinie durch einstellbare Ventile (64, 65) oder Widerstände variabel ist.

6. Hüftgelenkprothese nach einem der voranstehenden Ansprüche, **dadurch gekennzeichnet, dass** die Dämpfung in der Streckung konstant ist.

7. Hüftgelenkprothese nach einem der voranstehenden Ansprüche, **dadurch gekennzeichnet, dass** eine Energiespeichereinheit vorgesehen ist, die bei einer Streckung des Kunstbeines im Hüftgelenk Energie speichert und diese zur Unterstützung der Beugebewegung zumindest teilweise an das Kunstbein abgibt

8. Hüftgelenkprothese nach Anspruch 7, **dadurch gekennzeichnet, dass** als Energiespeichereinheit Federelement (7), insbesondere Kunststofffederelemente vorgesehen sind.

9. Verfahren zur Steuerung einer Hüftgelenkprothese mit zumindest einer Dämpfereinheit zur Dämpfung der Beuge- und Streckbewegung eines Kunstbeines, **dadurch gekennzeichnet, dass** die Dämpfung der Streckbewegung so eingestellt wird, dass ein Streckanschlag noch nicht erreicht wird, wenn bei einem normalen Gangmuster das Bein der contralateralen Seite schon angehoben wird und/oder in einen ersten Bewegungsabschnitt die Beugung nicht gedämpft wird, während eines zweiten Bewegungsabsahnittes die Dämpfung erhöht wird und in einem dritten Bewegungsabschnitt die Dämpfung konstant auf dem Endniveau des zweiten Bewegungsabschnittes gehalten wird.

10. Verfahren nach Anspruch 9, **dadurch gekennzeichnet, dass** als Steuerungsparameter der Beugewinkel, die Bewegungsrichtung und Geschwindigkeiten des Kunstbeines und/oder auf das Kunstbein ausgeübte Kräfte gemessen werden und in Abhängigkeit von den gemessenen Steuerungsparametern eine automatische Anpassung der Dämpfung erfolgt.

## Claims

1. A hip joint prosthesis (1) with connection means for securing to an upper securing device and to an artificial leg connected to the hip joint prosthesis as well as a control unit (6) is provided for controlling an extension movement in the hip joint and for controlling the step length, whereas the control unit (6) has at least one damper device (8, 64, 65, 91, 92, 94) for damping the flexion movement and extension movement in the hip joint **characterized in that** no damping is present in a first movement stage during flexion, the damping increases during a second movement stage, and, in a third movement stage, the damping remains constant at the end level of the second movement stage.

2. The hip joint prosthesis as claimed in claim 1, **characterized in that** the control unit (6) is designed as a hydraulic damper.

3. The hip joint prosthesis as claimed in claims I or 2, **characterized in that** the control unit (6) has a progressive damper characteristic curve.

4. The hip joint prosthesis as claimed in one of the preceding claims, **characterized in that** the control unit (6) is integrated in the hip joint.

5. The hip joint prosthesis as claimed in one of the preceding claims, **characterized in that** the damper characteristic curve can be varied via adjustable valves (64, 65) or resistances.

6. The hip joint prosthesis as claimed in one of the preceding claims, **characterized in that** the damping in extension is constant.

7. The hip joint prosthesis as claimed in one of the preceding claims, **characterized in that** an energy storage unit is provided which stores energy in the hip joint during an extension of the artificial leg and supplies at least some of this energy to the artificial leg in order to assist the flexion movement.

8. The hip joint prosthesis as claimed in claim 7, **characterized in that** spring elements (7), in particular plastic spring elements, are provided as the energy storage unit.

9. A method for controlling a hip joint prosthesis, with at least one damper unit for damping the flexion movement and extension movement of an artificial leg, **characterized in that** the damping of the extension movement is set in such a way that an extension limit is not yet reached when, during a normal gait pattern, the contralateral leg is already lifted and/or the flexion is not damped in a first movement stage, the damping is increased during a second movement stage, and, in a third movement stage, the damping is maintained constant at the end level of the second movement stage.

10. The method as claimed in claim 9, **characterized in that** the flexion angle, the direction of movement and speeds of the artificial leg and/or forces exerted on the artificial leg are measured as control parameters, and the damping is automatically adjusted as a function of the measured control parameters.

## Revendications

1. Prothèse d'articulation de hanche (1) comprenant des moyens de raccordement pour la fixation à un dispositif de fixation supérieur et à une jambe artificielle se raccordant à la prothèse d'articulation de hanche, et comprenant une unité de pilotage (6) pour le pilotage d'un mouvement d'extension dans l'articulation de hanche et d'une longueur de pas, l'unité de pilotage (6) présentant au moins un dispositif d'amortissement (8, 64, 65, 91, 92, 94) pour amortir le mouvement de flexion et d'extension dans l'articulation de hanche, **caractérisée en ce qu'**il n'y a pas d'amortissement pendant la flexion pour une première partie de mouvement, l'amortissement augmente pendant une deuxième partie de mouvement et dans une troisième partie de mouvement l'amortissement reste constant au niveau final de la deuxième partie de mouvement.

2. Prothèse d'articulation de hanche selon la revendication 1, **caractérisée en ce que** l'unité de pilotage (6) est formée par un amortisseur hydraulique.

3. Prothèse d'articulation de hanche selon la revendication 1 ou 2, **caractérisée en ce que** l'unité de pilotage (6) présente une caractéristique d'amortissement progressive.

4. Prothèse d'articulation de hanche selon l'une quelconque des revendications précédentes, **caractérisée en ce que** l'unité de pilotage (6) est intégrée dans l'articulation de hanche.

5. Prothèse d'articulation de hanche selon l'une quelconque des revendications précédentes, **caractérisée en ce que** la caractéristique d'amortissement est variable par l'Intermédiaire de résistances ou de soupapes (64, 65) réglables.

6. Prothèse d'articulation de hanche selon l'une quelconque des revendications précédentes, **caractérisée en ce que** l'amortissement est constant dans l'extension.

7. Prothèse d'articulation de hanche selon l'une quelconque des revendications précédentes, **caractérisée en ce qu'**il est prévu une unité d'accumulation d'énergie qui accumule l'énergie dans l'articulation de hanche lors d'une extension de la jambe artificielle et restitue celle-ci au moins partiellement à la jambe artificielle pour assister le mouvement de flexion.

8. Prothèse d'articulation de hanche selon la revendication 7, **caractérisée en ce que** des éléments élastiques (7) sont prévus en tant qu'unité d'accumulation d'énergie, en particulier des éléments élastiques en matière synthétique.

9. Procédé de pilotage d'une prothèse d'articulation de hanche comprenant au moins une unité d'amortissement pour amortir le mouvement de flexion et extension d'une jambe artificielle, **caractérisé en ce que** l'amortissement du mouvement d'extension est réglé de façon qu'une butée d'extension n'est pas encore atteinte lorsque dans un schéma de marche normal, la jambe du côté controlatéral est déjà soulevé et/ou dans une première partie de mouvement on n'amortit pas la flexion, pendant une deuxième partie de mouvement on augmente l'amortissement et dans une troisième partie de mouvement on maintient l'amortissement constant au niveau final de la deuxième partie de mouvement.

10. Procédé selon la revendication 9, **caractérisé en ce que** l'on mesure en tant que paramètres de pilotage l'angle de flexion, la direction de mouvement et des vitesses de la jambe artificielle et/ou des forces exercées sur la jambe artificielle, et une adaptation automatique de l'amortissement s'effectue en fonction des paramètres de pilotage mesurés.
